# EUROPÄISCHE PATENTANMELDUNG

(11) **EP 0 603 563 A1**
(43) Veröffentlichungstag der Anmeldung: **29.06.1994**
(21) Anmeldenummer: 93118878.3
(22) Anmeldetag: 24.11.1993
(51) Int. Cl.: A61B 1/12

(54) **Verfahren zur Prüfung und Reinigung von Endoskopen sowie Reinigungsgerät zur Durchführung des Verfahrens**

(30) Priorität: 04.12.1992 CH 3734/92
(71) Anmelder: F. GEHRIG + CO. AG, CH-6275 Ballwil (CH)
(72) Erfinder: Graf, Marcel, CH-6246 Altishofen (CH)
(74) Vertreter: Wagner, Wolfgang, Dr. Phil., Dipl.-Phys.

(57) **Zusammenfassung**

Bei der programmgesteuerten Reinigung von Endoskopen in einem Reinigungsgerät werden die Kopfteile in Druckkammern und zugleich die Einführungsschläuche in an dieselben anschliessenden Rohren in mehreren Reinigungsdurchgängen mit Reinigungsflüssigkeit, welche durch eine Umwälzpumpe (11) unter einem Druck von ca. 200 mbar eingeleitet wird, gereinigt, gespült, desinfiziert und reingespült. Vor jedem dieser Reinigungsdurchgänge wird durch den Druckprüfanschluss jedes Endoskops, der mittels eines Druckprüfadapters mit einem Druckluftanschluss (8a, 8b) des Reinigungsgeräts verbunden ist, von einem Druckluftaggregat (38) desselben erzeugte Druckluft eingeleitet, bis ein Innendruck von 250 mbar erreicht ist. Wird dieser Druck während 5 min nicht erreicht oder sinkt der Innendruck des Endoskops während einer anschliessenden Haltezeit von 30 sec um mehr als 30 mbar, wird die Umwälzpumpe (11) nicht in Gang gesetzt und die Reinigung abgebrochen. Andernfalls wird während des Reinigungsdurchgangs der Innendruck im Endoskop auf 250 mbar gehalten.

Das Endoskop wird auf diese Weise auf Dichtigkeit geprüft und vor einem Eindringen von Reinigungsflüssigkeit auch dann geschützt, wenn während der Reinigung ein Leck auftritt.

## Beschreibung

Die Erfindung betrifft ein Verfahren zur programmgesteuerten Prüfung und Reinigung von Endoskopen gemäss dem Oberbegriff des Anspruchs 1 sowie ein Reinigungsgerät zur Durchführung des Verfahrens.

Endoskope dienen zur Untersuchung des Magens oder Darms und weisen ein Kopfteil aus festem Material, gewöhnlich Kunststoff, auf mit einem Okular und gewöhnlich einem oder mehreren Anschlüssen und Knöpfen zur Auslösung bestimmter Funktionen des Endoskops sowie einen an das Kopfteil anschliessenden flexiblen Einführungsschlauch, welcher, von einem schlauchförmigen Kunststoffmantel geschützt, ein Glasfaserbündel enthält, das ein am distalen Ende angebrachtes Objektiv mit dem Okular verbindet sowie einen weiteren der Beleuchtung dienenden Lichtleiter und Kanäle, durch die Flüssigkeit abgesaugt, Wasser oder Luft eingeleitet und Werkzeug z. B. zur Entnahme von Gewebeproben eingeführt werden kann.

Mit dem Kopfteil ist gewöhnlich über einen Versorgungsschlauch, der ebenfalls, von einem Kunststoffmantel geschützt, mehrere Kanäle, die mit den Kanälen im Einführungsschlauch verbindbar sind, enthält, ein Versorgungsteil verbunden, an welchem besagte Kanäle sowie der der Beleuchtung dienende Lichtleiter in Anschlüsse zur Zuführung von Wasser, Luft und Licht sowie zum Absaugen von Flüssigkeit münden. Kopfteil, Einführungsschlauch, Versorgungsschlauch und Versorgungsteil sind von einer Hülle umgeben, welche aus den Gehäusen des Kopfteils und des Versorgungsteils und den Mänteln des Einführungs- und des Versorgungsschlauchs besteht und einen zusammenhängenden Innenraum umschliesst, welcher sowohl die erwähnten Kanäle und Lichtleiter als auch das empfindliche Okular enthält. Am Versorgungsteil ist auch ein Druckprüfungsanschluss angebracht, der eine Verbindung zum Inneren des Endoskops herstellt.

Die Reinigung von Endoskopen, die aus hygienischen Gründen sehr gründlich erfolgen und eine Desinfektion umfassen muss, ist äusserst heikel und umfasst gewöhnlich mehrere Reinigungsdurchgänge, bei denen mindestens der Einführungsschlauch einer unter Druck stehenden Reinigungsflüssigkeit ausgesetzt ist. Bei einem bekannten gattungsgemässen Verfahren (CH-A-675 064) wird daher das Kopfteil des Endoskops von einem schaumstoffgefüllten Gehäuse geschützt, in dessen Inneres Druckluft eingespeist wird. Dadurch kann nicht am Kopfteil Reinigungsflüssigkeit in das Endoskop eindringen. Falls jedoch der Einführungsschlauch ein Leck aufweist - und es ist vor allem das untere Ende desselben, das besonders leckanfällig ist - so kann dennoch Reinigungsflüssigkeit in das Innere des Endoskops eindringen und das Okular des teuren Instruments beschädigen. Ausserdem ist es nachteilig, dass das Kopfteil nicht gereinigt wird.

Es ist auch bekannt, vor der Reinigung mittels einer Handpumpe das Endoskop durch den Druckprüfungsanschluss aufzupumpen und auf diese Weise allfällige Lecks festzustellen. Diese Methode ist jedoch von vornherein nicht sehr zuverlässig - vor allem sind sehr kleine Lecks kaum feststellbar - und bietet keine Sicherheit gegen Lecks, die während der Reinigung auftreten oder sich vergrössern, was insbesondere bei der gewöhnlich erforderlichen Behandlung des Endoskops mit Reinigungsflüssigkeit höherer Temperatur leicht vorkommen kann. Ausserdem ist die Prüfung in diesem Fall ein von der Reinigung getrennter, verhältnismässig zeitraubender Vorgang.

Ein bewährtes gattungsgemässes Reinigungsgerät, bei dessen Verwendung jedoch vor der Reinigung eine Dichtigkeitsprüfung erforderlich ist, ist aus der Broschüre "Wasch- und Desinfektionsautomat für Endoskope SME 2000" der Firma Belimed AG, CH-5608 Stetten bekannt.

Aufgabe der Erfindung ist es, ein Verfahren zur integrierten Prüfung und Reinigung von Endoskopen anzugeben, bei welchem auch dann keine Gefahr besteht, dass Reinigungsflüssigkeit in das Innere des Endoskops eindringt, wenn sehr kleine, nicht feststellbare Lecks vorhanden sind und wenn Lecks im Verlauf der Reinigung auftreten oder sich vergrössern. Ausserdem soll ein geeignetes Reinigungsgerät zur Durchführung des Verfahrens angegeben werden.

Die Erfindung, wie sie in den Ansprüchen gekennzeichnet ist, schafft ein Verfahren, bei welchem die Prüfung zu Beginn und vorzugsweise vor jedem Reinigungsdurchgang erfolgt und die Reinigung bei Feststellung grösserer Lecks abgebrochen wird. Zudem wird das Endoskop gegen schädliche Auswirkungen kleinerer Lecks, wie sie auch während der Reinigung auftreten können, geschützt. Eine Beschädigung durch eindringende Reinigungsflüssigkeit kann somit mit grosser Sicherheit ausgeschlossen werden. Die Prüfung wird zusammen mit der Reinigung programmgesteuert durchgeführt. Sie erfordert kaum zusätzlichen Zeitaufwand und keine menschliche Arbeit oder Aufmerksamkeit. Im erfindungsgemässen Reinigungsgerät können beide Schritte automatisch ausgeführt werden.

Im folgenden wird die Erfindung anhand von nur ein Ausführungsbeispiel darstellenden Figuren näher erläutert.
Es zeigen
- Fig. 1a: ein erfindungsgemässes Reinigungsgerät in Vorderansicht, ohne Türe,

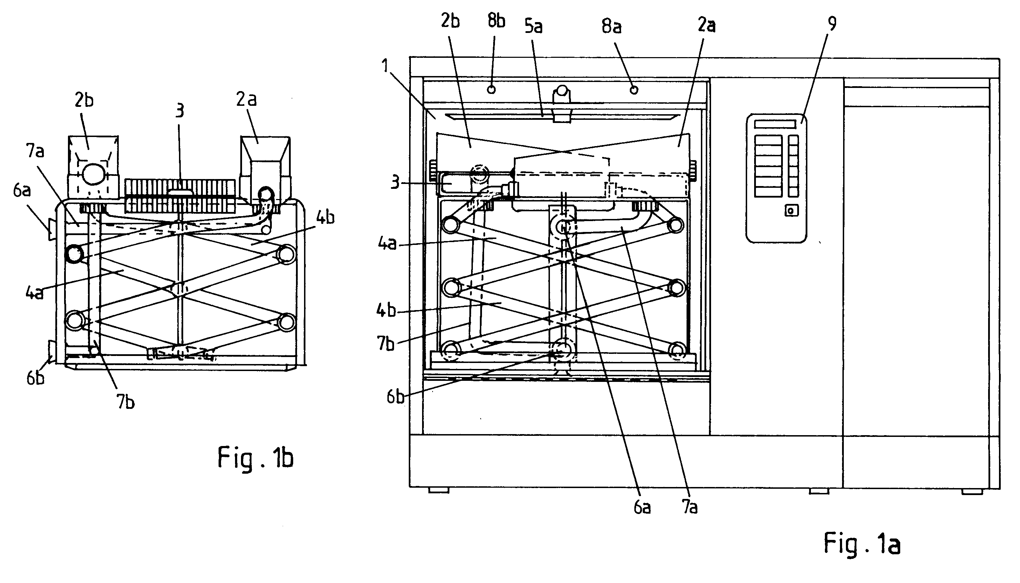

- Fig. 1b: Einbauten des Reinigungsgeräts nach Fig. 1a in Seitenansicht,
- Fig. 2: eine schematische Darstellung des Aufbaus des Reinigungsgeräts nach Fig. 1a, 1b,
- Fig. 3: schematisch einen Längsschnitt durch ein Endoskop in einem Reinigungsgerät nach Fig. 1a, 1b, 2 und
- Fig. 4: verschiedene Parameter als Funktionen der Zeit während der Durchführung des erfindungsgemässen Verfahrens.

Das Reinigungsgerät weist einen Reinigungsraum 1 auf, in welchem zwei Druckkammern 2a,b aus rostfreiem Stahl eingebaut sind, welche jeweils aus einem Unterteil und einem aufklappbaren Oberteil bestehen sowie ein Korb 3. An die Druckkammern 2a,b schliessen schraubenlinienförmige Rohre 4a,b aus Kunststoff an. In der Nähe der Decke und des Bodens des Reinigungsraums 1 sind Wascharme 5a,b drehbar aufgehängt. An der Rückwand sind zwei Druckwasseranschlüsse 6a,b angebracht, welche durch Rohre 7a,b mit den Druckkammern 2a,b verbunden sind sowie, unmittelbar unterhalb der Decke, Druckluftanschlüsse 8a,b. Neben der Oeffnung des Reinigungsraums 1 ist an der Vorderfront des Reinigungsgeräts eine Steuertafel 9 angeordnet.

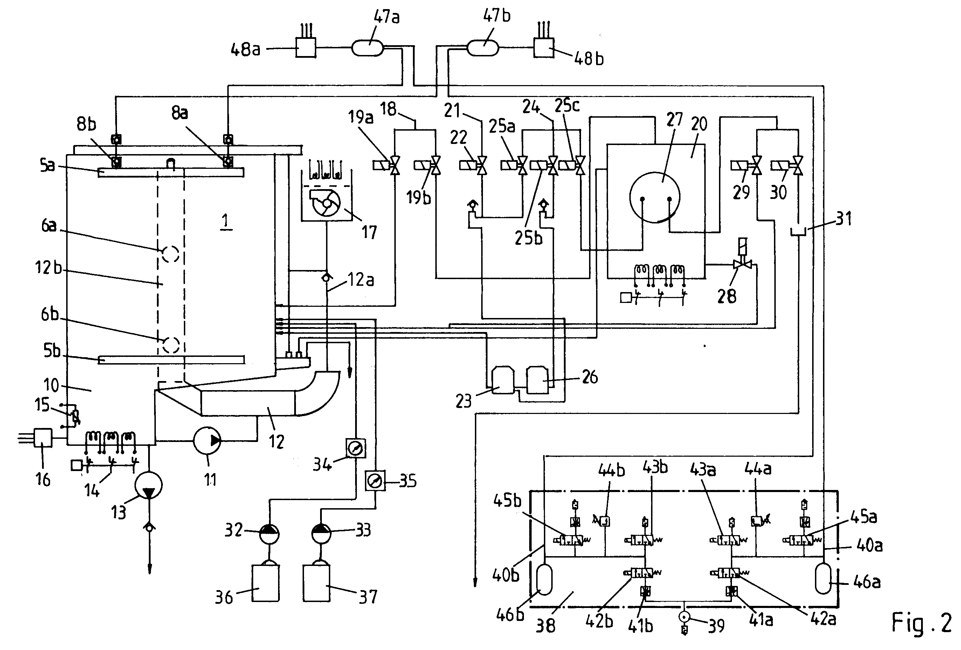

Wie aus Fig. 2 ersichtlich, wird der untere Teil des Reinigungsraums 1 von einem Tank 10 gebildet, welcher über eine Umwälzpumpe 11 mit einer Sammelleitung 12 verbunden ist, welche über einen ersten Zweig 12a die Wascharme 5a,b und über einen zweiten Zweig 12b die Druckwasseranschlüsse 6a,b versorgt. Durch eine Absaugpumpe 13 kann der Tank 10 entleert werden. Ausserdem ist er mit einer Heizung 14 und einem Temperaturfühler 15 versehen sowie mit einem Druckfühler 16 zur Ueberwachung seines Füllungsgrads. Mit dem ersten Zweig 12a der Sammelleitung 12 ist ein Heizgebläse 17 verbunden.

Das Reinigungsgerät wird über drei Anschlüsse mit Wasser versorgt. Ein Anschluss für entsalztes Wasser 18 ist über ein erstes Ventil 19a direkt mit dem Reinigungsraum 1 und über ein zweites Ventil 19b mit einem Boiler 20 verbunden. Ein Warmwasseranschluss 21 ist über ein Ventil 22 und einen Entkalker (Ionentauscher) 23 ebenfalls mit dem Reinigungsraum 1 verbunden, während ein Kaltwasseranschluss 24 über ein erstes Ventil 25a ebenfalls mit dem Entkalker 23, über ein zweites Ventil 25b mit einem demselben vorgeschalteten Regeneriersalzspeicher 26 und schliesslich über ein drittes Ventil 25c mit einem Wärmetauscher 27 in Verbindung steht, welcher im Boiler 20 angeordnet ist. Vom Boiler 20 und vom Wärmetauscher 27 führt je eine Leitung über ein Ventil 28 bzw. 29 in den Reinigungsraum 1, von ersterem ausserdem eine weitere über ein Ventil 30 zu einem Ablauf 31. Ebenfalls mit dem Reinigungsraum 1 verbunden sind, jeweils über eine Pumpe 32 bzw. 33 sowie einen Durchflussanzeiger 34 bzw. 35, ein Speicher 36 für Reiniger und eine Speicher 37 für Desinfektionsmittel.

Das Reinigungsgerät weist ausserdem ein Druckluftaggregat 38 auf mit einer Pumpe 39, welche über zwei Zweige die Druckluftanschlüsse 8a,b versorgt. Der erste Zweig weist, der Pumpe 39 nachgeschaltet, eine Leitung 40a auf, in welcher eine Drossel 41a und ein Einlassventil 42a liegen und von welcher ein Schnellentlüftungsventil 43a, ein mechanisches Ueberdruckventil 44a mit einem Ansprechdruck von 500 mbar, ein Drosselablassventil 45a und ein Ausgleichsspeicher 46a zum Ausgleich von Druckschwankungen abzweigen. Der zweite Zweig des Druckluftaggregats 38 ist mit einer Leitung 40b etc. genau gleich aufgebaut.

Die Leitung 40a führt zu einem mit dem Druckluftanschluss 8a verbundenen Druckluftspeicher 47a, welcher durch einen Drucksensor 48a überwacht wird, der das Einlassventil 41a steuert und so den Druck regelt. Die Leitung 40b ist analog über einen Druckluftspeicher 47b mit dem Druckluftanschluss 8b verbunden.

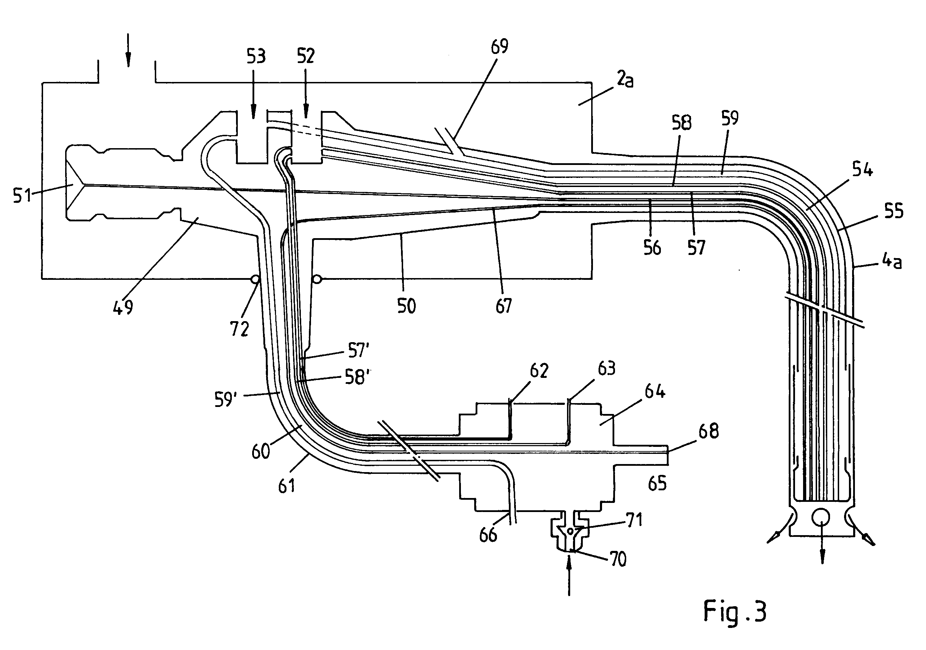

Das in Fig. 3 dargestellte Endoskop weist ein Kopfteil 49 mit einem starren Kunststoffgehäuse 50 auf, welches in der Druckkammer 2a liegt und ein Okular 51, ein empfindliches Bauteil, das mit Wasser oder sonstiger Reinigungsflüssigkeit nicht in Berührung kommen darf, enthält, sowie in Oeffnungen 52 bzw. 53 ein Luft-Wasser-Ventil und ein Absaugventil, welche zur Reinigung des Endoskops jedoch entfernt werden und daher nicht dargestellt sind. Am Kopfteil 49 ist ein flexibler Einführungsschlauch 54 befestigt, welcher im Rohr 4a liegt und, umhüllt von einem Kunststoffmantel 55, ein Glasfaserbündel 56 enthält, das ein Objektiv am distalen Ende des Einführungsschlauchs 54 mit dem Okular 51 verbindet, sowie einen Wasserkanal 57, einen Luftkanal 58 und einen Absaugkanal 59.

Der Wasserkanal 57 und der Luftkanal 58 münden in die Oeffnung 52, von welcher Fortsetzungen 57' und 58' dieser Kanäle ausgehen, die über einen flexiblen Versorgungsschlauch 60, der wie der Einführungsschlauch 54 von einem Kunststoffmantel 61 umhüllt ist, zu einem Wasseranschluss 62 und einen Luftanschluss 63 an einem Versorgungsteil 64 mit einem starren Kunststoffgehäuse 65 führen. Aehnlich münden der Absaugkanal 59 und seine Fortsetzung 59', die über den Versorgungsschlauch 60 zu einem Absauganschluss 66 am Versorgungsteil 64 führt, in die Oeffnung 53. Eine Lichtleitfaser 67 läuft von einem Lichtanschluss 68 am Versorgungsteil 64 über den Versorgungsschlauch 60, das Kopfteil 49 und den Einführungsschlauch 54 bis ans distale Ende des letzteren. Am Kopfteil 49 zweigt vom Absaugkanal 59 ein Anschluss 69 ab, durch welchen diverse Werkzeuge in den Absaugkanal und durch denselben an das distale Ende des Einführungsschlauchs 54 geführt werden können.

Der Kunststoffmantel 55 des Einführungsschlauchs 54, das Kunststoffgehäuse 50 des Kopfteils, der Kunststoffmantel 61 des Versorgungsschlauchs 60 und das Kunststoffgehäuse 65 des Versorgungsteils 64 umschliessen einen zusammenhängenden Innenraum des Endoskops, der, wie bereits erwähnt, auch das Okular 51 und andere empfindliche Teile enthält. Zur Dichtigkeitsprüfung ist daher am Kunststoffgehäuse 65 des Versorgungsteils 64 ein Druckprüfanschluss 70 vorgesehen mit einem Rückschlagventil 71, welches eine Verbindung ins Innere des Endoskops herstellt.

Zur Reinigung eines Endoskops wird der Einführungsschlauch 54 vollständig in das Rohr 4a eingeführt und das Kopfteil 49 in die Druckkammer 2a eingelegt. Der Versorgungsschlauch 60, genauer ein Anschlussstutzen für denselben am Kopfteil 49, wird durch eine mittels zweier Halbringe aus Gummi abgedichtete Durchführungsöffnung 72 aus zwei halbkreisförmigen Ausnehmungen im Oberteil und Unterteil der Druckkammer 2a dicht nach aussen geführt und in den Korb 3 gelegt. Dann wird die Druckkammer 2a geschlossen. Der Druckprüfanschluss 70 wird durch einen Druckprüfadapter, einen Kunststoffschlauch, der mittels eines Anschlussstücks an ihm befestigt wird, dicht mit dem Druckluftanschluss 8a verbunden. Ein zweites Endoskop wird in genau gleicher Weise in die zweite Druckkammer 2b eingeschlossen und mit dem Druckluftanschluss 8b verbunden. Anschliessend wird der Reinigungsraum 1 geschlossen.

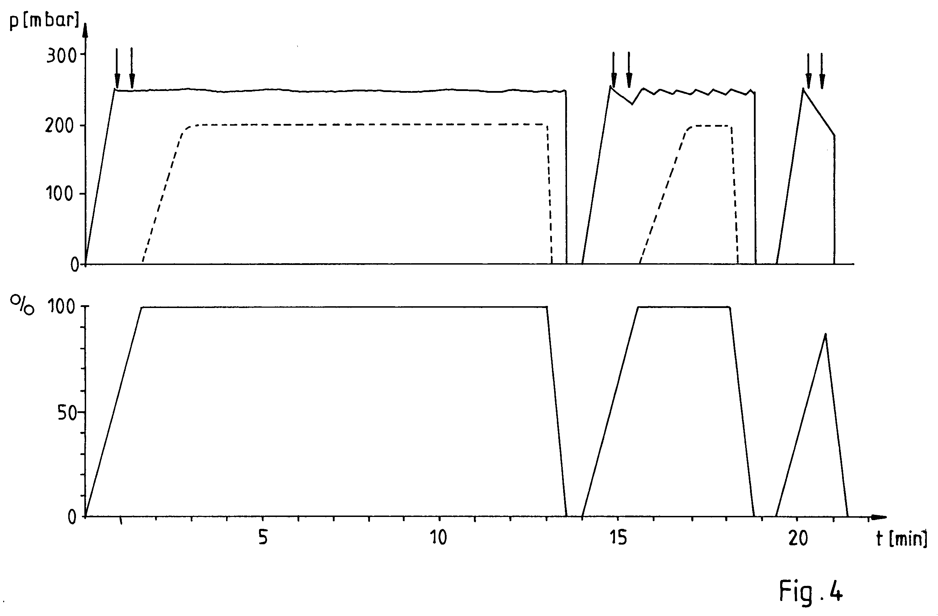

Zu Beginn der Reinigung wird nun durch den Anschluss 18 und das Ventil 19b entsalztes Wasser in den Boiler 20 geleitet und auf 85°C vorgeheizt. Anschliessend wird das Endoskop auf Dichtigkeit geprüft und der erste Reinigungsdurchgang, das eigentliche Reinigen, eingeleitet. In diesem Zusammenhang wird auf Fig. 4 verwiesen, wo unten der Füllungsgrad des Tanks 10 und oben der Innendruck des Endoskops (durchgezogen) und der Druck der Reinigungsflüssigkeit in der Druckkammer 2a (gestrichelt) als Funktionen der Zeit dargestellt sind. Während in den Tank 10 Warmwasser, das vom Warmwasseranschluss 21 über das Ventil 22 und den Entkalker 23 eingeleitet wird, was ca. 100 sec dauert, wird durch die Pumpe 39 über die Drossel 41a und das Einlassventil 42a der Druck im Druckluftspeicher 47a und damit auch im Endoskop auf einen Prüfdruck von 250 mbar erhöht. Nach einer Ausgleichszeit von etwa 5 sec wird der Luftdruck im Druckluftspeicher 47a festgestellt (erster Pfeil in Fig. 4). Nach 30 sec Haltezeit wird die Messung wiederholt (zweiter Pfeil). Die gleichen Messungen werden in der Druckluftkammer 47b, welche über den zweiten Zweig des Druckluftaggregats gleichzeitig auf einen Druck von 250 mbar gebracht wurde, ausgeführt. Falls in beiden Druckluftspeichern 47a,b der am Ende der Haltezeit gemessene Druck um weniger als einen Grenzwert von 30 mbar unter dem am Anfang derselben festgestellten Wert liegt, so wird das Endoskop als ausreichend dicht und damit das Prüfungsresultat als positiv betrachtet und der erste Reinigungsdurchgang begonnen durch Einschalten der Umwälzpumpe 11, während Reiniger aus dem Speicher 36 zudosiert wird, sodass sich die Druckkammern 2a,b mit Reinigungsflüssigkeit füllen. Die Anlage ist so ausgelegt, dass der Druck der Reinigungsflüssigkeit bei etwa 200 mbar liegt. Der Innendruck der Endoskope wird währenddessen durch die Drucksensoren 48a,b, welche die Einlassventile 42a,b steuern, auf 250 mbar ± 30 mbar gehalten. Dieser Druck wird von den Endoskopen gut vertragen, während bei Drücken oberhalb 300 mbar Beschädigungen nicht ausgeschlossen werden könnten.

Die Reinigungsflüssigkeit wird nun während ca. 10 min umgewälzt. Sie dringt jeweils in die Oeffnungen 52, 53 des Endoskops und spült den Wasserkanal 57, den Luftkanal 58 und den Absaugkanal 59 durch, an deren distalen Enden sie austritt und über das offene Ende des Rohrs 4a bzw. 4b in den Tank 10 zurückgeleitet wird. Desgleichen strömt Reinigungsflüssigkeit der Aussenseite des Einführungsschlauchs 54 entlang durch das Rohr 4a bzw. 4b, wodurch nicht nur die Kunststoffhülle 55 desselben gereinigt wird, sondern auch am distalen Ende des Einführungsschlauchs 54 durch den Strömungsabriss ein Unterdruck erzeugt wird, der die Druckdifferenz zu den Oeffnungen 52, 53 erhöht und eine gute Durchspülung der Kanäle bei verhältnismässig geringem statischem Druck bewirkt.

Aus den Oeffnungen 52, 53 strömt Reinigungsflüssigkeit auch durch die Fortsetzungen 57', 58' und 59' der vorerwähnten Kanäle und tritt am Wasseranschluss 62, am Luftanschluss 63 und am Absauganschluss 66 am Versorgungsteil 64 aus. Aeusserlich werden der Versorgungsschlauch 60 und das Versorgungsteil 64 durch die sich drehenden Wascharme 5a,b gereinigt, durch welche ebenfalls von der Umwälzpumpe 11 Reinigungsflüssigkeit gepumpt wird.

Gegen Ende des Reinigungsdurchgangs, währenddessen der Boiler 20 auf eine Desinfektionstemperatur von 93°C aufgeheizt wurde, wird die Umwälzpumpe 11 abgestellt und der Tank 10 durch die Absaugpumpe 13 entleert, während zugleich Druckluft aus den Druckluftspeichern 47a,b und damit den Endoskopen durch die Drosselablassventile 45a,b abgelassen und der Druck abgebaut wird.

Der nächste Reinigungsdurchgang, das spülen, läuft - einschliesslich der vorhergehenden Prüfung des Endoskops - prinzipiell völlig analog ab wie der erste, es wird jedoch kein Reiniger zugegeben und die Umwälzpumpe 11 läuft nur etwa 3 min.

Auch der nächste Reinigungsdurchgang, das Desinfizieren, dem auch eine Endoskopprüfung wie beschrieben vorangeht, unterscheidet sich nur in der Aufbereitung der Reinigungsflüssigkeit und der Dauer von den voraufgegangenen Durchgängen. Vom Kaltwasseranschluss 24 wird Kaltwasser über das Ventil 25c, den Wärmetauscher 27, wo es unter Abkühlung des entsalzten Wassers im Boiler 20 aufgewärmt wird und das Ventil 29 in den Tank 10 geleitet und Desinfektionsmittel aus dem Speicher 37 zudosiert. Während des Desinfizierens wird weiterhin Kaltwasser durch den Wärmetauscher 27 geleitet und über das Ventil 30 zum Ablauf 31, wodurch der Inhalt des Boilers 20 auf unter 55°C abgekühlt wird. Beim Desinfizieren läuft die Umwälzpumpe 11 während etwas mehr als 8 min.

Für den letzten Reinigungsdurchgang, das Reinspülen, wird das entsalzte Wasser aus dem Boiler 20, das ca. 10 min auf 93°C gehalten und so desinfiziert und dann auf unter 55°C abgekühlt wurde, da höhere Temperaturen von Endoskopen schlecht vertragen werden, über das Ventil 28 in den Tank 10 geleitet und während ca. 2 min umgewälzt. Ansonsten verläuft das Reinspülen einschliesslich der vorgängigen Endoskopprüfung genau wie die voraufgegangenen Reinigungsdurchgänge. Schliesslich werden die Endoskope durch vom Heizgebläse 17 ausgehende Heissluft getrocknet. Die Reinigung dauert etwa 40 min.

Selbstverständlich stellt die geschilderte Reinigung nur ein Beispiel dar. Das Reinigungsprogramm kann den Anforderungen und Umständen angepasst und variiert werden. Nur am prinzipiellen Ablauf eines Reinigungsdurchgangs mit vorhergehender Endoskopprüfung mittels Aufbau eines Innendrucks in demselben und anschliessender Umwälzung der Reinigungsflüssigkeit bei gleichzeitiger Aufrechterhaltung des über dem Druck der Reinigungsflüssigkeit liegenden Innendrucks ändert sich nichts.

Falls nun etwa das zu reinigende Endoskop in der Druckkammer 2b ein grosses Leck aufweist, so wird bereits vor dem ersten Reinigungsdurchgang der Prüfdruck von 250 mbar im Endoskop und damit im Druckluftspeicher 47b während eines Prüfintervalls von 5 min nicht erreicht. Die Umwälzpumpe 11 wird nicht in Betrieb gesetzt und die Reinigung abgebrochen. Auf der Steuertafel 9 erscheint eine Fehlermeldung.

In Fig. 4 ist nun der Fall dargestellt, dass im Endoskop, das in der Druckkammer 2a gereinigt wird, während der Reinigung ein Leck auftritt und sich vergrössert. Während des ersten Reinigungsdurchgangs, des eigentlichen Reinigens, ist das Endoskop noch dicht, nur gegen das Ende zeigen die grösser werdenden, aber immer noch deutlich unter 30 mbar liegenden Luftdruckschwankungen, dass das Endoskop Luft verliert und der Luftdruckspeicher 47a häufig nachgefüllt werden muss. Bei der Prüfung vor dem Spülen tritt dann während der Haltezeit von 30 sec ein deutlicher Druckabfall auf, der jedoch kleiner als der Grenzwert von 30 mbar ist. Dies deutet auf ein verhältnismässig kleines Leck hin. Die Druckverluste können vom Druckluftaggregat noch ohne weiteres ausgeglichen werden. Der Reinigungsdurchgang läuft normal ab, wobei jedoch der Druck im Druckluftspeicher 47a durch die Verluste und das Nachfüllen, das vom Drucksensor 48a, der das Einlassventil 42a steuert, ausgelöst wird, deutlich schwankt.

Bei der der Desinfektion voraufgehenden Prüfung tritt während der Haltezeit ein Druckabfall von mehr als 30 mbar ein. Dies deutet auf ein grösseres Leck hin und hat zur Folge, dass die Umwälzpumpe 11 nicht eingeschaltet und die Füllung des Tanks 10 abgebrochen wird. Er wird durch die Absaugpumpe 13 entleert. Die Druckluft wird über die Drosselablassventile 45a,b abgelassen und die Reinigung mit Fehlermeldung abgebrochen, ohne dass der Reinigungsdurchgang eingeleitet worden wäre.

## Patentansprüche

1. Verfahren zur programmgesteuerten Prüfung und Reinigung eines Endoskops mit einem Kopfteil (49) und einem an dasselbe anschliessenden Einführungsschlauch (54) sowie einem Druckprüfanschluss (70), wobei die Reinigung mindestens einen Reinigungsdurchgang umfasst, während dessen wenigstens der Einführungsschlauch (54) mindestens äusserlich einer unter Druck stehenden Reinigungsflüssigkeit ausgesetzt wird, **dadurch gekennzeichnet, dass** vor dem ersten Reinigungsdurchgang eine Prüfung des Endoskops durchgeführt wird, bei welcher über den Druckprüfanschluss (70) Druckgas in das Innere desselben geleitet und mindestens der Innendruck des Endoskops daraufhin überprüft wird, ob er innerhalb eines Prüfintervalls einen Prüfdruck erreicht und dass der Reinigungsdurchgang nur bei positivem Resultat der Prüfung ausgelöst wird und während desselben im Endoskop ein über dem Druck der Reinigungsflüssigkeit liegender Innendruck aufrechterhalten wird.

2. Verfahren nach Anspruch 1, **dadurch gekennzeichnet, dass** zur Prüfung des Endoskops gegebenenfalls, nachdem der Innendruck des Endoskops innerhalb des Prüfintervalls den Prüfdruck erreicht hat, ausserdem festgestellt wird, ob während eines Halteintervalls der Innendruck des Endoskops nicht ohne weitere Druckgaszufuhr um mehr als einen Grenzwert abnimmt.

3. Verfahren nach Anspruch 2, **dadurch gekennzeichnet, dass** während eines Reinigungsdurchgangs jeweils ein mindestens der Differenz zwischen dem Prüfdruck und dem Grenzwert entsprechender Innendruck im Endoskop aufrechterhalten wird und der Druck der Reinigungsflüssigkeit während des Reinigungsdurchgangs einen Maximaldruck nicht übersteigt, welcher kleiner ist als besagte Differenz.

4. Verfahren nach einem der Ansprüche 1 bis 4, **dadurch gekennzeichnet, dass** die Reinigung mehrere Reinigungsdurchgänge umfasst, welchen jeweils eine Prüfung des Endoskops vorausgeht.

5. Verfahren nach Anspruch 4, **dadurch gekennzeichnet, dass** bei den verschiedenen Reinigungsdurchgängen Reinigungsflüssigkeiten unterschiedlicher Temperaturen eingesetzt werden.

6. Verfahren nach einem der Ansprüche 1 bis 5, **dadurch gekennzeichnet, dass** der Prüfdruck zwischen 230 mbar und 300 mbar liegt.

7. Verfahren nach einem der Ansprüche 2 bis 6, **dadurch gekennzeichnet, dass** der Grenzwert zwischen 25 mbar und 50 mbar liegt.

8. Verfahren nach einem der Ansprüche 2 bis 7, **dadurch gekennzeichnet, dass** die Länge des Halteintervalls zwischen 20 sec und 60 sec liegt.

9. Reinigungsgerät zur Durchführung des Verfahrens nach einem der Ansprüche 1 bis 8, mit einem Tank (10), mit mindestens einer Druckkammer (2a, 2b) zur Aufnahme mindestens des Kopfteils (49) des Endoskops, sowie mit einer den Tank (10) mit der mindestens einen Druckkammer (2a, 2b) verbindenden Leitung und einer Umwälzpumpe (11) zum Pumpen von Reinigungsflüssigkeit durch dieselbe, **dadurch gekennzeichnet, dass** es für jede Druckkammer (2a, 2b) einen von einer steuerbaren Druckgaseinheit versorgten Druckgasanschluss zur Verbindung mit dem Druckprüfanschluss (70) des Endoskops aufweist.

10. Reinigungsgerät nach Anspruch 9, **dadurch gekennzeichnet, dass** der Tank (10) den unteren Teil eines Reinigungsraums (1) bildet, in welchem auch die mindestens eine Druckkammer (2a, 2b) angeordnet ist.

11. Reinigungsgerät nach Anspruch 10, **dadurch gekennzeichnet, dass** die mindestens eine Druckkammer (2a, 2b) eine abdichtbare Durchführungsöffnung (72) aufweist zur Durchführung eines Versorgungsschlauchs (60) des Endoskops, welcher das Kopfteil (49) mit einem Versorgungsteil (64), welches den Druckprüfungsanschluss (70) trägt, verbindet und dass der Druckgasanschluss ausserhalb der Druckkammer (2a, 2b) im Reinigungsraum (1) angeordnet ist.

12. Reinigungsgerät nach einem der Anschlüsse 9 bis 11, **dadurch gekennzeichnet, dass** an die mindestens eine Druckkammer (2a; 2b) ein am Ende zum Tank (10) hin offenes Rohr (4a; 4b) zur Aufnahme des Einführungsschlauchs (54) des Endoskops anschliesst.
